# EUROPEAN PATENT SPECIFICATION

(11) **EP 3 709 306 B1**
(45) Date of publication and mention of the grant of the patent: **25.08.2021**
(21) Application number: 19162869.2
(22) Date of filing: 14.03.2019
(51) Int. Cl.: G16H 40/40, A47K 5/12, G01R 19/165, G01R 31/3835

(54) **TECHNIQUE FOR DETECTING OPERATION EVENTS OF AN AUTOMATIC DISPENSER**
VERFAHREN ZUM ERFASSEN VON BETRIEBSEREIGNISSEN EINES AUTOMATISCHEN SPENDERS
TECHNIQUE PERMETTANT DE DÉTECTER DES ÉVÉNEMENTS D'OPÉRATION D'UN DISTRIBUTEUR AUTOMATIQUE

(43) Date of publication of application: 16.09.2020
(73) Proprietor: GWA Hygiene GmbH, 18435 Stralsund (DE)
(72) Inventor: WALZ, Marcel, 18437 Stralsund (DE); GEBHARDT, Tobias, 18119 Rostock (DE)
(74) Representative: Röthinger, Rainer

(56) References cited:
- US-A1- 2015 199 865
- US-A1- 2018 024 202

## Description

### Technical Field

The present disclosure relates to the field of automatic dispensers. In particular, a sensor unit configured to detect operation events of an automatic dispenser and a system comprising the sensor unit and the automatic dispenser are presented. A method for detecting operation events of an automatic dispenser and a computer program product are presented also.

### Background

In recent years, there has been a rise in the number of new infections incurred inside hospitals. Especially patients are affected by this phenomenon. The main cause for these new infections is suspected to be an incorrect or improper use of hand hygiene products, such as fluid disinfectants, also due to the rising workload of the employees of the hospitals.

A suitable countermeasure could be a comprehensive electronic recording of the usage of hand hygiene products. However, until now, such an approach is not easily possible for some kinds of dispensers. For example, the monitoring of an automatic dispenser already attached to a wall of a patient room is technically difficult as the monitoring function has to be retrofitted.

Conventional sensor units monitor the operation of an automatic dispenser based on measurements of a weight of a disinfectant fluid acting on the sensor or based on a movement of a pumping arm of the dispenser. An example of a sensor unit of the latter type is given in WO 2018/053627 A1, wherein an actuation of the dispenser is measured by a vibration sensor. Another example is presented in US 2011/0121974 A1, wherein a movement or vibration of the dispenser is associated with an actuation of the dispenser.

Further prior art is known from the documents US 2018/0024202 A1 and US 2015/0199865 A1. The first of these documents (US 2018/0024202 A1) discloses systems and methods for detection and analysis of battery condition information of, for example, battery powered hand hygiene product dispensers. The battery condition information may be used to identify a variety of information concerning the dispenser. The second of these documents (US 2015/0199865 A1) provides techniques and/or systems for evaluating dispenser functionality of a dispenser for dispensing a material. In one example, current measurements are taken during a dispense event and evaluated to determine whether a mechanical problem exists.

It has been found that the use of vibration sensors to monitor dispenser operation can lead to erroneous measurements. Such erroneous measurements can, for example, occur as a result of shocks acting on the dispenser from the outside.

### Summary

There is a need for a sensor unit that can be used with automatic dispensers for reliably detecting operation events of said dispensers.

The present invention is defined by the sensor unit of claim 1 and the method of claim 16. Further developments are set out in the dependent claims.

According to one aspect, a sensor unit configured to detect operation events of an automatic dispenser is provided. The sensor unit comprises a detection unit configured to detect a change in an operating voltage of the dispenser and a controller. The controller is configured to analyse the voltage change for a predetermined characteristic and, based on the analysis, assign the voltage change to an operation event of the dispenser. The controller is further configured to create a dataset indicative of the operation event.

The sensor unit may be an independent assembly that can handled separately from the automatic dispenser. As such, the sensor unit may be usable with different kinds of automatic dispensers. The sensor unit may be configured to be connected to the automatic dispenser during its initial installation process, or may be retrofitted to an already installed dispenser. In other variants, the sensor unit may be integrated into the automatic dispenser at a manufacturing site.

The detection unit may be configured to measure the operating voltage of the dispenser. The detection unit may further be configured to create a digital signal representative of said voltage. The operating voltage may be continuously measured by the detection unit, or the detection unit may measure the operating voltage at pre-defined points in time. Alternatively or additionally, the detection unit may comprise a comparing unit configured to compare the measured operating voltage to a pre-defined threshold and to trigger the detection unit to create the digital voltage signal if the operating voltage is above the threshold.

The predetermined characteristic may comprise at least one of the following: an initial voltage before the voltage change, an end voltage after the voltage change, a voltage offset between an initial voltage and an end voltage, an amplitude of the voltage change (i.e., the voltage change "as such"), an indication whether the voltage change is a voltage decrease or increase (i.e., a "direction" of the voltage change), a duration of the voltage change, and a rate of the voltage change.

In some variants, the end voltage may be defined as the voltage that would be measured as the initial voltage of an immediately following dispensing operation. In such or other variants, any voltage recovery processes may not yet be fully completed when the end voltage is measured. In some variants, the amplitude of the voltage change may be defined as the largest difference between the initial voltage and any other voltage value measured temporally after the initial voltage and before the end voltage.

In some variants, the detection threshold may be defined so as to take into account one or more of the following effects upon detection of operation events: a (possibly changing) charging level of a power source of the dispenser, a (possibly variable) recovery time of the power source of the dispenser between dispensing events, a specific characteristic of the power source of the dispenser, environmental influences (temperature, humidity, air pressure), defects of the dispensing mechanism and software defects.

The controller may be configured to create an indication dependent on a result of the comparison. The indication may, for example, indicate that the comparison of the predetermined characteristic with the at least one detection threshold associated with the predetermined characteristic yields a negative result. A negative result may be a voltage change above a maximum detection threshold and/or below a minimum detection threshold. The indication may comprise at least one of a value of the voltage change violating the detection threshold, a value of the detection threshold, a predetermined characteristic of the voltage change, an associated operation event, and a time stamp. The indication may be included in the data set.

The controller may be configured to modify the at least one detection threshold based on one or multiple detected voltage changes that have previously been assigned to an operation event. As an example, the controller may modify the detection threshold by comparing the voltage changes assigned to the same operation event during successive voltage measurements and identify a specific trend in the measurements. If, for example, the measurements successively get closer to the threshold, the threshold may be moved farther away so as to maintain a certain safety margin. Additionally, or in the alternative, if the measurements successively move farther away from the threshold, the threshold may be moved closer to the measurements.

The operation event type may further comprise at least one of the following event types: a start of a dispensing event, a recovery process of the dispenser after a dispensing event, a recovery time of the power supply of the dispenser after actuation, a replacement of the dispenser, and an opening of a cover of the dispenser.

The controller may be configured to include one or more of the following items of information in the data set: an operation event type, a time stamp, a voltage offset between an initial voltage and an end voltage of the voltage change, an amplitude of the voltage change, a duration of the voltage change, an indication as discussed above, and a battery level of the dispenser in the data set.

The sensor unit may further comprise a data interface configured to output the dataset (e.g., to a portable storage device or to a remote processing unit). The data interface may be configured to output the data set wirelessly or via a wired connection. A wireless data output may for example be performed via Bluetooth, Wireless LAN (WLAN), ZigBee, Radio Frequency Identification (RFID) or Near Field Communication (NFC). A wire-based data output may for example be performed via a data cable or a portable storage device.

The sensor unit may further comprise a detection interface configured to connect the sensor unit to a power source of the dispenser. The sensor unit may be connected to the power source of the dispenser remotely or locally. When connected locally, the sensor unit may be located in a housing of the dispenser. In this case, the detection interface may comprise wires, a connection clip and/or an inductive and/or capacitive element. When connected locally, the detection interface may comprise one of a male and a female plug directly coupled to a plug of the opposite type at the side of the dispenser. When connected remotely, the sensor unit may be located outside a housing of the dispenser (e.g., using a wired connection between the sensor unit and the power source of the dispenser). The detection interface may be electrically connected to the detection unit of the sensor unit, in order to enable the detection unit to detect a voltage change.

Power may be supplied to the sensor unit from a power source of the dispenser. In this case, the detection interface may be configured to source power from the power source of the dispenser to the sensor unit. Additionally, or in the alternative, the sensor unit may comprise a power source configured to supply power to the sensor unit. The power source may comprise one or both of a rechargeable battery and a solar cell module.

The sensor unit may further comprise a signaling unit configured to signal to a user an operation event and/or additional information. The signaling unit may comprise at least one of an optoelectronic module and an acoustic exciter.

According to a further aspect, a system is provided comprising the sensor unit presented herein and an automatic dispenser to which the sensor unit is coupled so as to be configured to detect a change in an operating voltage of the dispenser.

The automatic dispenser may be a fluid dispenser, such as a dispenser for fluid disinfectants. The automatic dispenser may be configured to be wall-mounted. The dispenser may be a dispenser that detects the presence of a user (e.g., by using an infrared sensor) and, based on said detection, automatically dispenses a fluid, for example using a motor-operated pumping mechanism. The dispenser may comprise a power source, for example batteries or a solar cell module, for providing electrical power to the pumping mechanism. The power source may be accessible by a user for replacement and/or reparation purposes.

The dispenser may be a disinfectant dispenser, a soap dispenser, a hand care dispenser or a cleaning agent dispenser. The dispenser may be configured to dispense a fluid substance or a non-fluid substance or article.

According to a further aspect, a method for detecting operation events of an automatic dispenser is provided. The method comprises detecting a change in an operating voltage of the dispenser and analysing the voltage change for a predetermined characteristic. The method further comprises assigning the voltage change to an operation event of the dispenser based on a comparison of the predetermined characteristic with at least one detection threshold associated with the predetermined characteristic, and creating a dataset associated with the operation. According to the method, multiple operation event types are defined for categorizing operation events, wherein each operation event type is associated with at least one detection threshold. The method further comprises assigning the operation event to a particular operation event type based on the at least one predetermined characteristic and the at least one detection threshold associated with this operation event type. The operation event type comprises an activation of the dispenser.

The method may be performed by the sensor unit presented herein. As such, the method may comprise one or more further steps as described above and below. According to a still further aspect a computer program product is provided comprising program code portions for performing the steps of the method presented herein when the computer program product is executed on one or more processors.

### Brief Description of the Drawings

Further details, advantages and aspects of the present disclosure will become apparent from the following description of embodiments taken in conjunction with the drawings, wherein:
- Fig. 1A: shows a perspective front view of an embodiment of a sensor unit connected to an automatic dispenser;
- Fig. 1B: shows a perspective rear view of the sensor unit of Fig. 1A with an opened housing;
- Fig. 2A: shows a perspective rear view of the sensor unit of Fig. 1 connected to a power source of the automatic dispenser according to a first connection method;
- Fig. 2B: shows a perspective side view of a second embodiment of a sensor unit connected to a power source of an automatic dispenser according to a second connection method;
- Fig. 3: shows an exemplary time dependency of an operating voltage of an automatic dispenser;
- Fig. 4: shows the time dependency of Fig. 3 with several exemplary detection thresholds;
- Fig. 5: shows the determination of exemplary event parameter values for the time dependency of Fig. 3;
- Fig. 6: shows an exemplary dataset created by a controller of the sensor unit and being indicative of operation event types; and
- Fig. 7: shows a flow diagram of an exemplary method performed by a sensor unit for detecting operation events of a dispenser.

### Detailed Description

In the following description, exemplary embodiments of a sensor unit configured to detect operation events of an automatic dispenser, a system comprising the sensor unit and the dispenser, as well as a method for detecting operation events of an automatic dispenser will be explained with reference to the drawings. The same reference numerals will be used to denote the same or similar structural features.

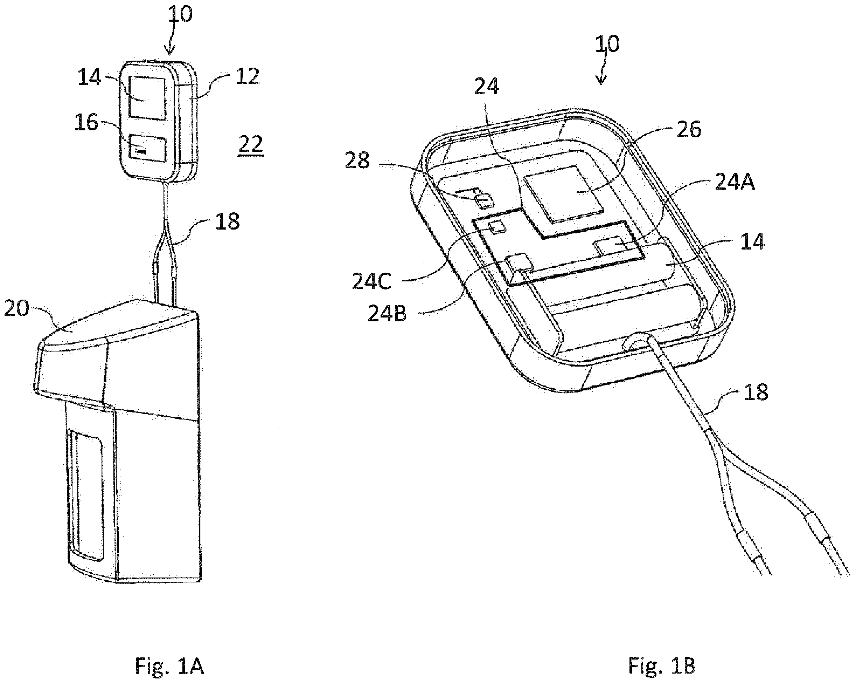

Fig. 1A shows a perspective front view of a first embodiment of a sensor unit 10. The sensor unit 10 comprises a housing 12, a power source 14, a signaling unit 16 and a detection interface 18. The sensor unit 10 is connected to an automatic dispenser 20. The dispenser 20 is configured to detect the presence of a user and dispense, based on the detection, a fluid, such as a disinfectant, by using a motor-operated pumping mechanism (not shown). In the embodiment depicted in Fig. 1A, the sensor unit 10 and the dispenser 20 are mounted to a wall 22.

The power source 14 is optional and comprises a solar cell module. The solar cell module 14 is configured to supply power to the sensor unit 10. Alternatively, or in addition, the power source 14 may comprise a battery pack. The battery pack may be rechargeable and may be connected to the solar cell module. In a still further variant, the sensor unit 10 sources power from the dispenser 20.

The signaling unit 16 comprises an optoelectronic module, e.g., a Light Emitting Display (LED) or a Liquid Crystal Display (LCD), configured to provide optical information to a user. Alternatively or additionally, the signaling unit 16 may comprise an acoustic exciter (e.g., a speaker) configured to provide acoustic information to a user.

Fig. 1B shows a perspective rear view of the sensor unit 10, wherein the rear part of the housing 12 has been removed. A detection unit 24, a controller 26, a data interface 28 and a portion of the detection interface 18 are arranged inside the housing 12.

In this embodiment, the detection unit 24 comprises an optional voltage converter 24A, a comparator 24B and an analog-to-digital-converter (ADC) 24C. The voltage converter 24A is electrically connected to the detection interface 18 and configured to convert a higher input voltage to a lower output voltage or vice versa. The comparator 24B is configured to send a signal (e.g., a wake-up signal or an activation signal) to the ADC 24C when an input voltage of the comparator 24B exceeds a voltage threshold. The ADC 24C is configured to measure a voltage, e.g., received from the voltage converter 24A or from the detection interface 18 directly. The ADC 24C is further configured to convert the measured voltage into a digital signal representative of the voltage change and to transmit the digital signal to the controller 26.

The controller 26 is configured to receive and process the digital signal from the detection unit 24 and to create one or more data sets. The controller 26 may for example be a microcontroller (µC), a field programmable gate array (FPGA) or a system on a chip (SoC). The controller 26 may be coupled to a memory (not shown) of the sensor unit 10 for storing the datasets.

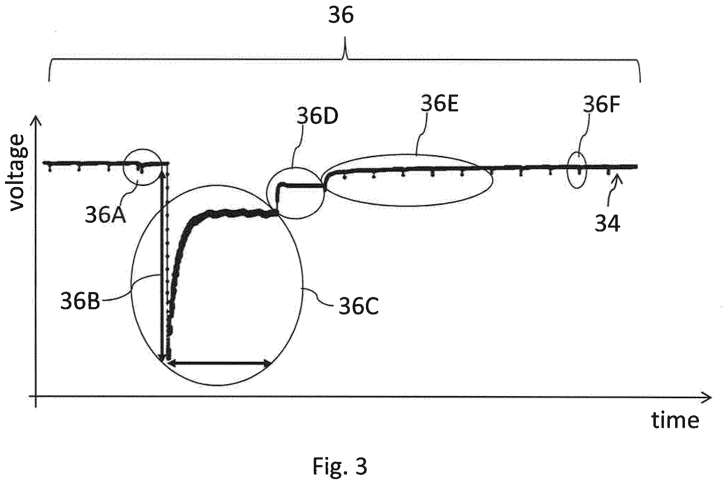

The controller 26 is configured to analyze changes in the digital signal received from the ADC 24C for a predetermined characteristic, to assign the signal change to an operation event of the dispenser based on the analysis, and to create a dataset indicative of the operation event. The operation of the controller 26 will be described in more detail with reference to Figs. 3 to 5 below.

The data interface 28 is configured to output datasets created by the controller 26 for processing by a remote processing unit (not shown). The processing unit may for example be comprised by a data hub of a hand hygiene compliance system of a hospital. The hand hygiene compliance system may be configured to monitor the usage of disinfectants throughout a hospital. In the embodiment of Fig. 1B, the data interface 28 is configured to transmit the datasets in a wireless manner, e.g., via WLAN. In an alternative embodiment, the datasets may be transmitted by the data interface 28 (e.g., via a Universal Serial Bus (USB) port) to a portable storage device coupled to the sensor unit 10. The storage device (not shown) may then be connected to the processing unit in order to transmit the data sets stored on the storage device to the processing unit.

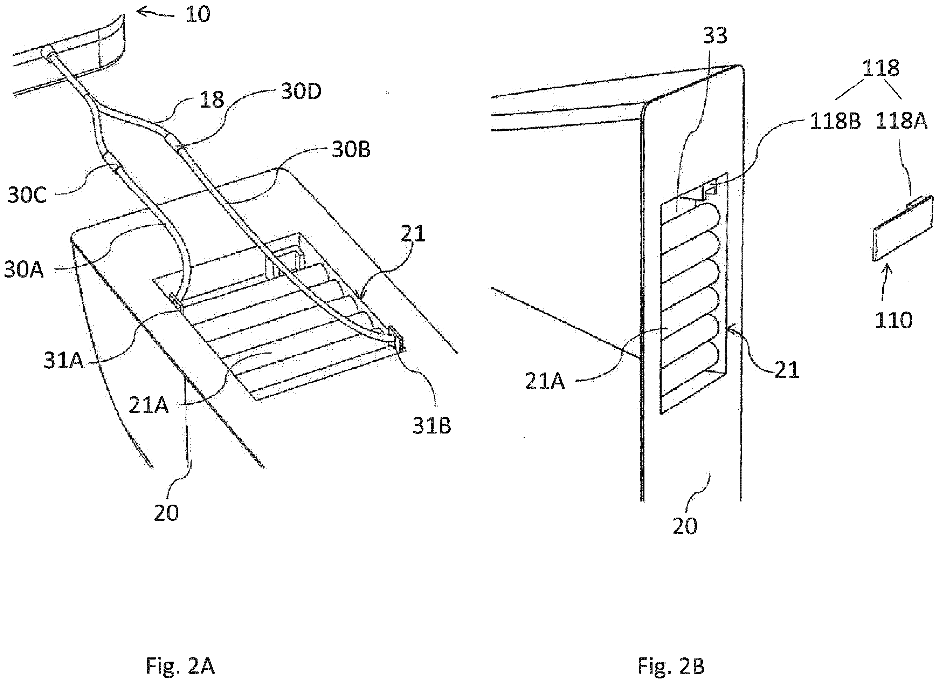

The detection interface 18 is configured to connect the sensor unit 10, and in particular the detection unit 24, to a power source of the dispenser 20, either directly (e.g., locally) or indirectly (e.g., remotely). Solutions for connecting the sensor unit 10 to the dispenser 20 via the detection interface 18 will now be described with reference to Figs. 2A and 2B.

Figs. 2A and 2B show different variants of electrically connecting the sensor unit 10, 110 to the dispenser 20. Fig. 2A shows a first embodiment, wherein the sensor unit 10 is located remotely from the dispenser 20 (see Fig. 1A) and electrically connected to a power source 21 of the dispenser 20 via wires 30A, 30B. Fig. 2B shows a second embodiment, wherein a sensor unit 110 is located in a housing of the dispenser 20 and electrically connected to the power source 21 via male and female connectors 118A, 118B.

The power source 21 is depicted in Figs. 2A and 2B to comprise batteries 21A. It is to be understood that the power source 21 may comprise a variety of other elements, such as a transformer coupled to an external power source, such as a wall socket.

By electrically connecting the sensor unit 10 to the power source 21, the detection unit 24 (e.g., the ADC 24C) is able to measure a voltage of the power source 21, which in the following will be referred to as "operating voltage of the dispenser". In the embodiment depicted in Fig. 2A, the detection interface 18 is electrically connected to wires 30A, 30B and connection clips 31A, 31B. The connection clips 31A and 31B are respectively electrically connected to a positive and a negative terminal of the power source 21. The wires 30A and 30B are likewise electrically connected to the clips 31A and 31B, respectively. In the embodiment depicted in Fig. 2A, the detection interface 18 comprises quick exchange plugs 30C and 30D configured to electrically connect the sensor unit 10 to the wires 30A and 30B. The quick exchange plugs 30C and 30D enable a quick exchange of the sensor unit 10 and/or the dispenser 20 without the need of disconnecting the wires 30A, 30B from the clips 31A, 31B.

The detection interface 18 illustrated in Fig. 2A provides an easy way for connecting the sensor unit 10 to the dispenser 20. Moreover, the sensor unit 10 can be connected to already installed dispensers 20, i.e., may be retrofitted thereto. Hence, the usage of automatic dispensers initially not equipped with voltage sensing capabilities can be monitored in an easy way by retrofitting them with the sensor unit 10 as described above.

In the embodiment depicted in Fig. 2B, the sensor unit 110 is co-located with and electrically connected to a power source 21 of a dispenser 20 via a detection interface 118. In this embodiment, the sensor unit 110 does not comprise a power source 14 and a signaling unit 16. The detection interface 118 comprises a male plug 118A on the side of the sensor unit 110 and a female plug 118B on the side of the dispenser 20. In this embodiment, the sensor unit 110 can be fully inserted into a recess 33 of the power source 21. Furthermore, electrical power can be directly provided from the power source 21 to the sensor unit 110 via the detection interface 118. Thus, the overall size of the sensor unit 110 can be reduced as no additional power source of the sensor unit 110 is needed.

In the scenario illustrated in Fig. 2B, the sensor unit 10, 110 may be connected to the dispenser 20 by a manufacturer of the dispenser 20. In other words, newly manufactured dispensers may already be equipped with a sensor module 10, 110.

Fig. 3 shows an embodiment of a time dependency 34 of an operating voltage of an automatic dispenser 20. The operating voltage is measured by the detection unit 24 and analyzed by the controller 26 for a predetermined characteristic. In the embodiment of Fig. 3, six exemplary types of voltage changes 36A to 36F with specific predetermined characteristics are marked and will be explained in detail in the following.

The first voltage change type 36A comprises besides one of multiple regularly appearing power drops an additional larger power drop. Thus, an exemplary predetermined characteristic of the voltage change 36A would be a voltage change rate (i.e., the first temporal derivative of the operating voltage) changing its sign twice within a short period of time, with an end voltage being smaller than an initial voltage.

The second voltage change type 36B comprises a sudden and significant drop of the operating voltage. Thus, an exemplary characteristic of the voltage change 36B would be a voltage decrease having a large amplitude over a certain period of time. During the detection of the third voltage change type 36C, the operating voltage continuously increases. An exemplary characteristic of the voltage change 36C would therefore be a voltage increase (i.e., an overall positive sign of a voltage change rate).

The fourth voltage change type 36D comprises an almost instantaneous jump of the operating voltage to a specific value. Thus, an exemplary characteristic of the fourth voltage change type 36D would be the amount of the voltage increase (i.e., its amplitude).

During the detection of the fifth voltage change type 36E, the operating voltage slightly increases. Hence, an exemplary characteristic of the voltage change 36E would be a minimal voltage offset between an initial voltage and an end voltage during a longer period of time.

The sixth voltage change type 36F is a brief voltage drop. Therefore, an exemplary characteristic would be a change rate of the operating voltage changing its sign twice during a short period of time.

Each predetermined characteristic can be defined by a value of the voltage change (i.e., a voltage amplitude) and an associated time duration. As an example, during detection of voltage change 36B, the operating voltage drops during 50 ms by 500 mV. As a further example, during detection of voltage change 36D, the operating voltage increases by 100 mV during 200 ms.

For the measured time dependency of the operating voltage, at least one detection threshold can be predefined. The at least one detection threshold may be modified during operation of the dispenser 20.

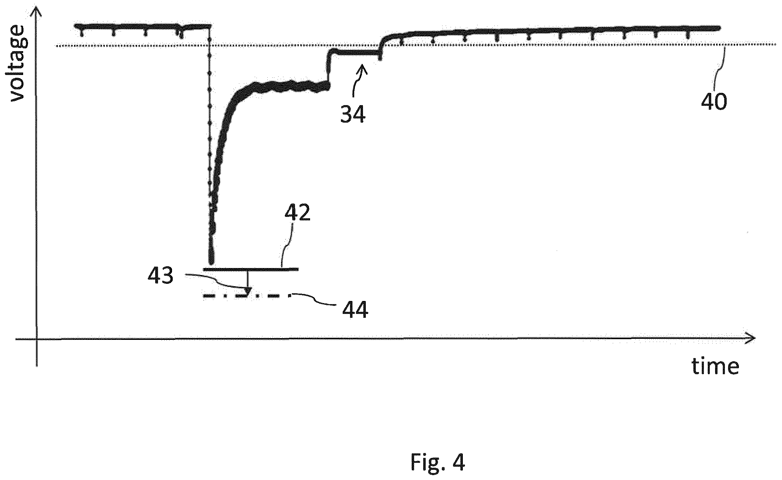

Fig. 4 shows two exemplary types of detection thresholds for the time dependency 34 illustrated in Fig. 3. In Fig. 3, a first detection threshold 40 and a second detection threshold 42 are depicted. The first detection threshold 40 (depicted in Fig. 4 by a dotted line) defines a first limit for evaluating the measured operating voltage further. An operation voltage change that remains above the first detection threshold 40 would not be considered to comprise an operation event. The second detection threshold 42 (depicted in Fig. 4 by a solid line) defines a second limit for detecting a specific operation event of the dispenser 20. An operating voltage falling below the second detection threshold 42 would not be assigned to an actuation event of the dispenser 20.

The detection thresholds may be defined to take into account one or more of the following circumstances that may have an influence on properly detecting an operation event: a possibly changing charging level of a power source of the dispenser 20, a recovery time of the power source of the dispenser 20 between dispensing events, a specific characteristic of the power source of the dispenser 20 (e.g., to cover different dispenser types), changing environmental influences (temperature, humidity, air pressure), defects of the dispensing mechanism and software defects. A software defect may for example be determined upon detecting a substantially continuous actuation of the dispenser 20, e.g., 60 actuations per minute over a time period of 10 minutes. Such a detected behavior of the dispenser 20 is not plausible and may thus be associated with a software defect (e.g., of the firmware) of the dispenser 20.

If the predetermined characteristic of a detected voltage change satisfies the detection thresholds 40, 42 (i.e., falls below the detection threshold 40 but remains above the detection threshold 42), the controller 26 assigns the voltage change to an operation event. The assignment is based on the insight that the power produced by the power source 21 of the dispenser 20 is directly related to the operating voltage of the power source 21 by the formula ΔW=ΔU^{∗}Q, wherein Q defines the electrical charge of the power source. In other words, a change in the operating voltage ΔU of the power source 21 is to be understood as an amount of energy ΔW sourced from the power source 21 to perform operation events of the dispenser (e.g., for actuation of an electrically-operated pump).

With reference again to Fig. 3, the exemplary voltage change types 36A to 36F are assigned to specific operation events of the dispenser as follows.

The predetermined characteristic of the voltage change 36A is caused by an operation event of the dispenser which is short in time and only requires a minimum amount of energy. A typical example of such kind of operation event would be the activation of a sensor of the dispenser 20, e.g., an infrared sensor that detects the presence of a user's hands and will lead to a dispensing event. Thus, the voltage change 36A is indicative an activation of the dispenser, but will as such not be considered further since it remains above the threshold 40.

The characteristic voltage change 36B is caused by an operation event consuming a significant amount of energy, such as the start of an electric motor of the dispenser 20. The voltage change 36B satisfies the detection thresholds 40, 42 (i.e., falls below the detection threshold 40 but remains above the detection threshold 42). Hence, the controller 26 assigns the voltage change 36B to the start of a dispensing event on the time axis.

The voltage change 36C is caused by an operation event lasting a longer time span, but requiring a fewer amount of energy than the start of an electric motor of the dispenser 20. Typically, these conditions are fulfilled during an actual pumping event, that is the electric motor repeatedly moving a pumping piston or other mechanical member of the dispenser 20. Therefore, the controller 26 assigns the voltage change 36C to a dispensing event over a certain time span that started with detection of voltage change 36B.

During the voltage change 36D, the electric motor consumes fewer energy over a shorter time span than during the voltage change 36C. This voltage characteristic may occur when the actual dispensing event is finished and the pumping mechanism returns to its initial position. Therefore, the controller 26 assigns the voltage change 36D to a recovery process of the dispenser 20 after a dispensing event. Start of voltage change 36D may mark the end of the time span that started with detection of voltage change 36B.

The time behaviour of the voltage change 36E is associated with an operation event during which the electric motor of the dispenser 20 is switched off and consumes no further energy. This is typically the case during a recovery step of the power source 21 after a dispensing event. Hence, the controller 26 assigns the voltage change 36E to a recovery time of the power source 21 of the dispenser 20 after actuation. Thus, the controller 26 assigns the voltage change 36F the end of a dispensing event.

Besides the regularly appearing voltage peaks 36F, no further change is measured after voltage change 36E. This behaviour indicates that the power source 21 of the automatic dispenser 20 is not loaded at all.

It is to be understood that the voltage changes 36A to 36F depicted in Fig. 3, their predetermined characteristics as well as the associated operation events assigned thereto are purely explanatory and should in no way restrict the scope of the present disclosure.

The controller 26 is configured to modify, or adjust, one or both of detection thresholds 40, 42 based on multiple detected voltage changes that have previously been assigned to operation events. This adjustment is exemplarily shown for detection threshold 42 in Fig. 4.

According to one implementation, a table storing pre-set detection thresholds 40, 42 is stored in a memory of the sensor unit 10, 110. The pre-set minimum and/or maximum voltage values may be updated continuously as will now be described in more detail. Additionally, each specific voltage change 36 assigned to an operation event is stored as a data set in the memory. The controller 26 is configured to compare the voltage change 36 assigned to a specific operation event (e.g., the voltage change 36B assigned to the start of a dispensing event) in the current voltage measurement with a voltage change 36 assigned to the same specific operation event in former voltage measurements, (i.e., the voltage change assigned to the start of a dispensing event in multiple previous measurements). Based on the comparison, the controller 26 calculates a trend for the measured voltage values of the specific operation event (e.g., the start of a dispensing event). For example, if a trend for the voltage decrease of voltage change 36B shows a tendency to get substantially continuously closer to the detection threshold 42 during several voltage measurements, the controller 26 adjusts the detection threshold 42 in the direction of arrow 43 towards the adjusted detection threshold 44, representing a lower minimum value of the operating voltage. As another example, the controller 26 adjusts the detection threshold 40 to a higher minimum value for performing voltage measurements if the trend for the power drop of voltage change 36B shows a tendency to move continuously farther away from the detection threshold 40 during several voltage measurements.

It is to be understood that the adjusted detection threshold 44 is taken in consideration when assigning the voltage changes to operation events. For example, the adjusted detection threshold 44 may replace the detection threshold 42. That is, the predetermined characteristic of a voltage change is then compared to the detection thresholds 40 and 44.

As the detection thresholds 40, 42 may constantly be adjusted, the sensor unit 10, 110 can be used with a variety of different dispensers and/or on a variety of different locations. For example, the charging level of a dispenser's power source may vary among different dispensers. Moreover, different dispensers may be equipped with different power sources, e.g., different kinds of batteries showing different electrochemical behaviours. As a further example, the environmental conditions, such as temperature, humidity and air pressure may vary between different locations of the sensor unit. However, as the detection thresholds 40, 42 are constantly adjusted based on former measurements, these disturbing effects to the measurement can be quickly recognized and covered by the detection thresholds 40, 42.

In one variation, the controller 26 is configured to create an indication of a comparison of a predetermined characteristic of a particular voltage change with at least one of the detection thresholds 40, 42. For example, a predetermined characteristic (e.g., amplitude of voltage change 36B) may satisfy detection threshold 40 but violate detection threshold 42. The indication may be stored in a memory of the sensor unit 10 (e.g., in a data set). The indication may for example mark an operation event that has violated a detection threshold. The indication may be associated, for example in a data set, with the voltage level of the violated detection threshold, the voltage value by which the detection threshold was violated and a timestamp. Alternatively, or additionally, the controller 26 may adjust the violated detection threshold 40, 42 after the indication has been created.

On the other hand, keeping the detection thresholds 40, 42 unchanged is advantageous in a case in which the sensor unit 10, 110 is always operated with the same dispenser 20 and on the same location. In this case, it can be assumed that for example the environmental conditions and the electrochemical behaviour of the power source of the dispenser only vary slightly. Hence, the pre-set detection thresholds 40, 42 already cover these disturbances and constantly adjusting the detection thresholds 40, 42 would provide no further measurement accuracy. Additionally, as the detection unit 24 is already precisely calibrated, it is very likely that a voltage measurement violating the detection thresholds 40, 42 should in fact be considered as an error, rather than adjusting the detection thresholds 40, 42 based on this measurement.

The controller 26 is further configured to define multiple operation event types for categorizing operation events. Each operation event type is associated with at least one detection threshold 40, 42. For example, an operation event having predetermined characteristics that satisfy both detection thresholds 40, 42 is assigned to the operation event type "actuation". As a further example, an operation event violating the detection threshold 42 is assigned to the operation event type "error".

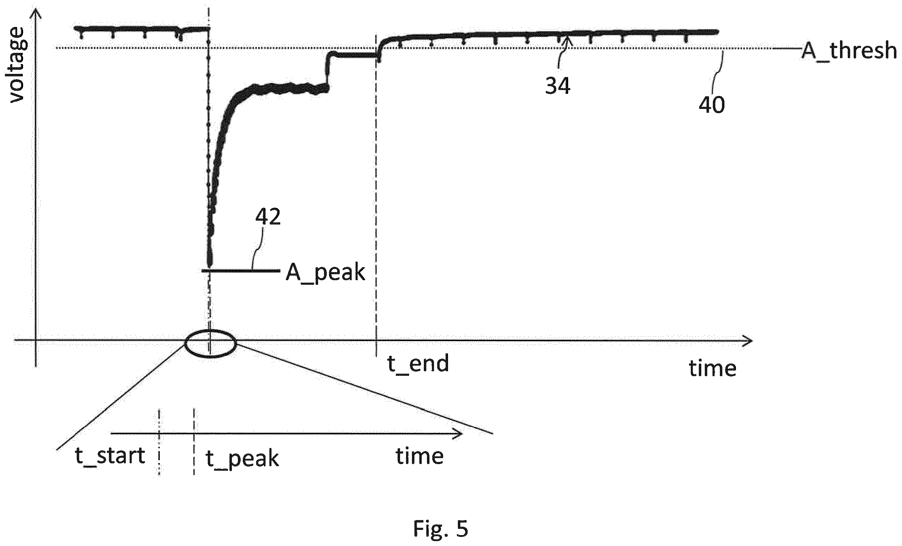

A specific operation event type may be characterized by specific event parameters, such as a time stamp of the event, a time duration of the event and/or a voltage change (e.g., an amplitude) of the event. Exemplary values of event parameters for the operation event "actuation" as discussed above, i.e., an operation event having predetermined characteristics that satisfy both detection thresholds 40, 42, are shown in Fig. 5.

In Fig. 5, the operation event "actuation" comprises the voltage changes 36B to 36D of Fig. 3. A parameter denoted t_start in Fig. 5 represents the beginning of the operation event "actuation", in particular the beginning of the voltage change 36B. The parameter t_start is the point in time where the representation of the time dependency 34 falls below the first detection threshold 40. At point in time t_start, the detected voltage exceeds a threshold amplitude A_thresh that equals the value of the first detection threshold 40.

The start of the actual pumping event (i.e., the start of voltage change 36C) is denoted by t_peak. This is the point in time where the representation of the time dependency 34 increases after the sudden drop during the voltage change 36B. At t_peak, the sign of the derivative of the representation of the time dependency 34 changes from negative to positive. Additionally, or in the alternative, the point in time t_peak may be derived as by the global minimum of the representation of the time dependency 34. Said global minimum may be also calculated using the first temporal derivative of the representation of the time dependency 34 (either numerically or analytically).

Knowing t_peak, the amplitude A_peak of the operating voltage at the start of the actual pumping event may be determined. For example, A_peak be read out from a memory in which the representation of the time dependency 34 is stored (e.g., in the form of tuples [measured voltage; time]) by looking up the measured voltage at the point in time corresponding to t_peak.

The point in time t_end represents the end of the operation event type "actuation" and is the point in time where the representation of the time dependency 34 rises above the first detection threshold 40 again, i.e., above the value A_thresh. The time duration of the operation event "actuation" is given by the difference between t_start and t_end. Likewise, the voltage change (i.e., the amplitude) of the operation event type "actuation" is given by the difference between A_thresh and A_peak. Furthermore, t_peak may be used as a time stamp of the operation event "actuation". It is to be understood that the depicted event parameters (time duration, voltage change and time stamp) are purely explanatory and are in no way considered to restrict the present disclosure.

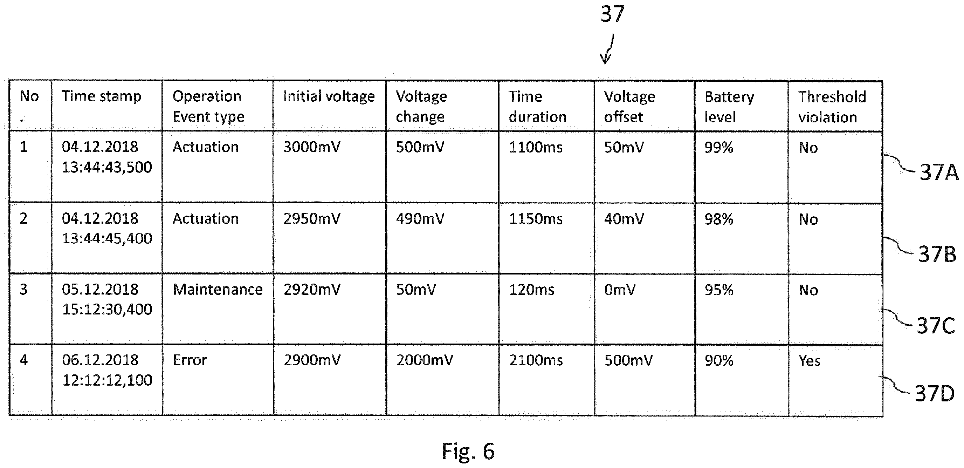

After the controller 26 has identified an operation event of a particular operation event type, the controller 26 creates a dataset. Fig. 6 shows an exemplary table 37 with four exemplary data sets 37A to 37D created by the controller 26 and being indicative of different operation event types.

As can be seen from Fig. 6, the table 37 indicates for each dataset 37A to 37D (i.e., for each detected operation event) a consecutively assigned operation event type number, a time stamp, a category of the operation event type, an initial voltage of the operation event, a voltage change (amplitude) during the detection of the operation event, a duration of the operation event type, a voltage offset between initial and end voltage of the operation event type, a battery level of the dispenser and an indication whether the voltage change assigned to the operation event type violates a detection threshold. The time stamp, the amplitude during the detection of the operation event type and the duration of the operation event type may be calculated as described in conjunction with Fig. 5 above.

Fig. 6 shows that operation events 1 and 2 comprise a voltage decrease (amplitude) of about 500 mV that satisfies both detection thresholds 40, 42. Therefore, the operation events 1 and 2 are both assigned to the operation event type "Actuation".

Operation event 3 shows a voltage decrease of 50 mV, a time duration of 120 ms and no offset. This operation event is assigned to an operation event type "Maintenance", indicative, for example, of the opening of a cover of the dispenser 20.

The voltage decrease of event number 4 is 2000 mV, lasts 2100 ms and also shows a large offset of 500 mV. These characteristics are unusual for a normal operating dispenser. Since the detection threshold 42 has been violated, this operation event is assigned to the operation event type "Error".

The table 37 may be stored in a storage medium (not shown) of the sensor unit 10, 110. Such an approach permits comparing different data sets 37A to D with one another. This comparison has already been described above with regard to adjusting the detection thresholds 40, 42. Alternatively or additionally, the table 37 may be transmitted to a processing unit via the data interface 28, as also described above. Therefore, the usage of an automatic dispenser 20 equipped with the sensor unit 10, 110 can be monitored by a central health care monitoring system.

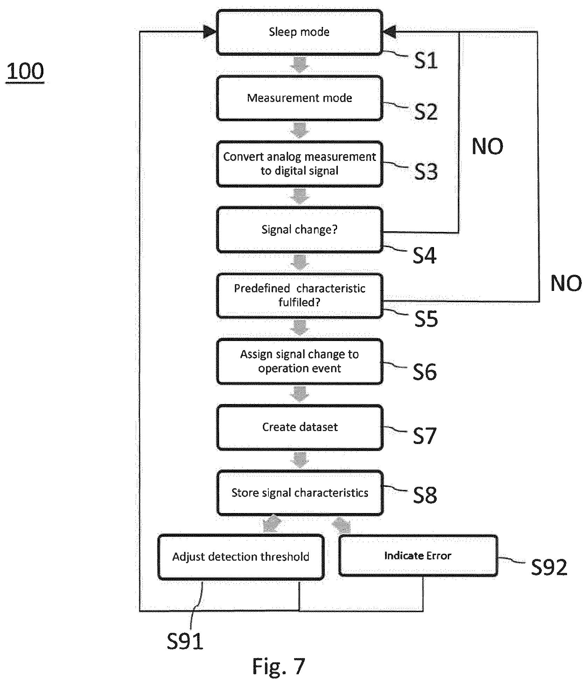

Fig. 7 shows a flow diagram 100 of an exemplary method performed by a sensor unit 10, 110 connected to a power source 21 of an automatic dispenser 20 for detecting operation events of the dispenser 20.

At the beginning, the sensor unit 10, 110 is waken up from a sleep mode (step S1) in order to perform a measurement of an operating voltage of the dispenser 20. For example, the controller 26 may be configured to send a wake-up signal at predefined points in time to the detection unit 24 (e.g., in fixed time intervals). Alternatively or additionally, the comparator 24B may be configured to trigger a wake-up signal to the ADC 24C if a predefined threshold of the operating voltage is trespassed.

In step S2, the detection unit 24 changes to a measurement mode for measuring the operating voltage of the power source 21 of the dispenser 20.

The actual measurement of the operating voltage starts in step S3, wherein the ADC 24C measures the operating voltage of the power source 21, creates a digital signal representative of the operating voltage and transmits the signal to the controller 26.

In step S4, the controller 26 analyses the signal received from the ADC 24C for a change of a voltage signal. If no voltage signal change is detected in step S4, the sensor unit 10, 110 returns to the sleep mode. In the case of a detected voltage signal change, the controller 26 analyses in step S5 the voltage change for a predetermined characteristic as described with reference to Figs. 3 and 4 above.

The controller 26 then selectively assigns the detected signal change to an operation event of the dispenser 20 in step S6 and creates in table 37 a new dataset indicative of the operation event in step S7 (see Fig. 6).

As described with reference to Fig. 4, the controller 26 stores the signal characteristics (e.g., the predetermined characteristic and a trend change rate for operation events) on a storage medium of the sensor unit 10, 110 in step S8.

The controller 26 may adjust the pre-set detection thresholds based on one or more (including the last) voltage measurements (step S91) or creates an error indication if the voltage change assigned to an operation event violates the pre-set detection thresholds (step S92). The sensor unit 10, 110 then returns to the sleep mode.

Analysing the operation of an automatic dispenser 20 based on its operating voltage provides for a high accuracy. Even a slight voltage change is precisely detectable by voltage sensors, therefore enabling a very exact analysis of the operation of the dispenser 20. Moreover, voltage sensors are widely available at low costs. Thus, the production costs of a sensor unit 10, 110 as described herein are low.

Additionally, automatic dispensers 20 are often mounted to walls and are thus subject to external shocks. For example in a hospital very often patient beds or heavy medical equipment is passing close to the wall-mounted dispenser 20, creating strong vibrations. If the operation of such a dispenser 20 is analysed based on mechanical parameters, e.g., the movement of a pumping arm or the vibration of an motor-operated pumping mechanism, the measurement of these parameters will be negatively influenced by the external vibrations. However, the operating voltage of the power source 21 of the dispenser 20 is not affected by external vibrations.

While exemplary realisations of a sensor unit and a system comprising the same have been described, it will be understood that the sensor unit and the system can be modified in many ways. Therefore, the present disclosure is only limited by the claims appended hereto.

## Claims

1. A sensor unit (10, 110) configured to detect operation events of an automatic dispenser (20), the operation events comprising dispensing events, the sensor unit (10, 110) comprising:
a detection unit (24) configured to detect a sequence of changes (36A-D) in an operating voltage of the dispenser (20); and
a controller (26) configured to:
- analyse the voltage changes (36A-D) for predetermined characteristics to detect a voltage change (36C) that is related to a dispensing event of the dispenser (20);
- assign one of the detected voltage changes (36B, 36C, 36D) to a dispensing event of the dispenser (20) based on the analysis,
wherein a second voltage change (36B) preceding a third voltage change (36C) that is related to the dispensing event is indicative of a start of an electric motor of the dispenser (20) and is assigned to a start of the dispensing event based on a voltage decrease having an amplitude falling below a first detection threshold (40) over a certain period of time as a predetermined characteristic,
wherein the third voltage change (36C) related to the dispensing event lasts a longer time span and requires a fewer amount of energy than the preceding second voltage change (36B) and is assigned to the dispensing event during which the electric motor repeatedly moves a mechanical member of the dispenser (20) based on a continuous increase of the voltage as a predetermined characteristic, and
wherein the time span of the third voltage change (36C) related to the dispensing event ends with detection of a following fourth voltage change (36D) during which the electric motor consumes fewer energy over a shorter period of time than during the third voltage change (36C) related to the dispensing event, wherein the following fourth voltage change (36D) is assigned to a recovery process of the dispenser (20) based on an amplitude of the voltage increase as a predetermined characteristic; and
- create a dataset (37) indicative of the dispensing event and the time span of the third voltage change (36C) related to the dispensing event.

2. The sensor unit (10, 110) according to claim 1, wherein
the predetermined characteristic comprises at least one of the following:
- an initial voltage before one of the voltage changes (36A-D),
- an end voltage after one of the voltage changes (36A-D),
- a voltage offset between an initial voltage and an end voltage,
- an amplitude of one of the voltage changes (36A-D),
- an indication whether the voltage change is a voltage decrease or increase,
- a duration of one of the voltage changes (36A-D), and
- a rate of one of the voltage changes (36A-D).

3. The sensor unit (10, 110) according to claim 1 or 2, wherein
the controller (26) is configured to assign one of the voltage changes (36A-D) to one of the operation events based on a comparison of the predetermined characteristic with at least one detection threshold (40, 42, 44) associated with the predetermined characteristic.

4. The sensor unit (10, 110) of claim 3, wherein
the controller (26) is configured to create an indication dependent on a result of the comparison, wherein, as an option, the indication is included in the data set (37).

5. The sensor unit (10, 110) according to claim 3 or 4, wherein
the controller (26) is configured to modify the at least one detection threshold (40, 42, 44) based on one or multiple of the detected voltage changes (36A-D) that have previously been assigned to one of the operation events.

6. The sensor unit (10, 110) according to any of claims 3 to 5, wherein
multiple operation event types are defined for categorizing operation events,
wherein each operation event type is associated with at least one detection threshold (40, 42, 44),
and wherein the controller (26) is configured to assign the operation event to a particular operation event type based on the at least one predetermined characteristic and the at least one detection threshold (40, 42, 44) associated with this operation event type.

7. The sensor unit (10, 110) according to claim 6, wherein
the operation event type comprises at least one of the following:
- an activation of the dispenser (20),
- a start of a dispensing event,
- a recovery process of the dispenser (20) after a dispensing event,
- a recovery time of the power supply (21) of the dispenser (20) after actuation,
- a replacement of the dispenser (20),
- an opening of a cover of the dispenser (20), and
- a change in the power supply (21) of the dispenser (20).

8. The sensor unit (10, 110) according to any of the preceding claims, wherein
the controller (26) is configured to include at least one of the following items of information in the data set (37): an operation event type, a time stamp, a voltage offset between an initial voltage and an end voltage of one of the voltage changes (36A-D), an amplitude of one of the voltage changes (36A-D), and a battery level of the dispenser (20).

9. The sensor unit (10, 110) according to any one of the preceding claims, comprising
a data interface (28) configured to output the dataset (37) to a processing unit.

10. The sensor unit (10, 110) according to any of the preceding claims, comprising
a detection interface (18) configured to connect the sensor unit (10, 110) to a power source (21) of the dispenser (20).

11. The sensor unit (10, 110) according to any of the preceding claims, wherein
power is supplied to the sensor unit (10, 110) from a power source (21) of the dispenser (20).

12. The sensor unit (10, 110) according to any of the preceding claims, comprising
a power source (14) configured to supply power to the sensor unit (10, 110).

13. The sensor unit (10) according to any of the preceding claims, further comprising
a signalling unit (16) configured to signal to a user one of the operation events and/or additional information.

14. A system comprising
the sensor unit (10, 110) according to any of the preceding claims; and
an automatic dispenser (20) to which the sensor unit (10, 110) is coupled so as to be configured to detect a change in an operating voltage of the dispenser (20).

15. The system according to claim 14, wherein
the dispenser (20) is a disinfectant dispenser, a soap dispenser, a hand care dispenser or a cleaning agent dispenser.

16. A method for detecting operation events of an automatic dispenser (20), the operation events comprising dispensing events, the method comprising:
- detecting (S4) a sequence of changes (36A-D) in an operating voltage of the dispenser (20);
- analysing (S5) the voltage changes (36A-D) for predetermined characteristics to detect a voltage change (36C) that is related to a dispensing event of the dispenser (20);
- assigning (S6) one of the detected voltage changes (36B, 36C, 36D) to a dispensing event of the dispenser (20) based on the analysis,
wherein a second voltage change (36B) preceding a third voltage change (36C) that is related to the dispensing event is indicative of a start of an electric motor of the dispenser (20) and is assigned to a start of the dispensing event based on a voltage decrease having an amplitude falling below a first detection threshold (40) over a certain period of time as a predetermined characteristic,
wherein the third voltage change (36C) related to the dispensing event lasts a longer time span and requires a fewer amount of energy than the preceding second voltage change (36B) and is assigned to the dispensing event during which the electric motor repeatedly moves a mechanical member of the dispenser (20) based on a continuous increase of the voltage as a predetermined characteristic, and
wherein the time span of the third voltage change (36C) related to the dispensing event ends with detection of a following fourth voltage change (36D) during which the electric motor consumes fewer energy over a shorter period of time than during the third voltage change (36C) related to the dispensing event, wherein the following fourth voltage change (36D) is assigned to a recovery process of the dispenser (20) based on an amplitude of the voltage increase as a predetermined characteristic; and
- creating (S7) a dataset (37) indicative of the dispensing event and the time span of the third voltage change (36C) related to the dispensing event.

17. A computer program product comprising program code portions for performing the steps of the method according to claim 16 when the computer program product is executed on one or more processors.

## Patentansprüche

1. Sensoreinheit (10, 110), die eingerichtet ist, um Betriebsereignisse eines automatischen Spenders (20) zu erfassen, wobei die Betriebsereignisse Spendeereignisse umfassen, und wobei die Sensoreinheit (10, 110) umfasst:
eine Erfassungseinheit (24), die eingerichtet ist, um eine Folge von Änderungen (36A-D) in einer Betriebsspannung des Spenders (20) zu erfassen; und
eine Steuerung (26), die eingerichtet ist, um:
- die Spannungsänderungen (36A-D) hinsichtlich vorbestimmter Merkmale zu analysieren, um eine Spannungsänderung (36C) zu erfassen, die mit einem Spendeereignis des Spenders (20) zusammenhängt;
- eine der erfassten Spannungsänderungen (36B, 36C, 36D) einem Spendeereignis des Spenders (20) auf Grundlage der Analyse zuzuordnen,
wobei eine zweite Spannungsänderung (36B), die einer dritten mit dem Spendeereignis zusammenhängenden Spannungsänderung (36C) vorausgeht, einen Start eines Elektromotors des Spenders (20) angibt und einem Start des Spendeereignisses auf der Grundlage einer Spannungsabnahme, die eine Amplitude aufweist, die innerhalb einer bestimmten Zeitspanne unter einen ersten Erfassungsschwellenwert (40) fällt, als ein vorbestimmtes Merkmal zugeordnet ist,
wobei die dritte, mit dem Spendeereignis zusammenhängende Spannungsänderung (36C) eine längere Zeitspanne andauert und eine geringere Energiemenge benötigt als die vorhergehende zweite Spannungsänderung (36B) und dem Spendeereignis, bei dem der Elektromotor wiederholt ein mechanisches Element des Spenders (20) bewegt, auf der Grundlage eines kontinuierlichen Anstiegs der Spannung als ein vorbestimmtes Merkmal zugeordnet ist, und
wobei die Zeitspanne der dritten Spannungsänderung (36C), die mit dem Spendeereignis zusammenhängt, mit der Erfassung einer nachfolgenden vierten Spannungsänderung (36D) endet, während welcher der Elektromotor über einen kürzeren Zeitraum weniger Energie verbraucht als während der dritten Spannungsänderung (36C), die mit dem Spendeereignis zusammenhängt, wobei die nachfolgende vierte Spannungsänderung (36D) einem Erholungsprozess des Spenders (20) auf der Grundlage einer Amplitude des Spannungsanstiegs als ein vorbestimmtes Merkmal zugeordnet ist; und
- einen Datensatz (37) zu erzeugen, der das Spendeereignis und die Zeitspanne der dritten Spannungsänderung (36C), die mit dem Spendeereignis zusammenhängt, angibt.

2. Sensoreinheit (10, 110) nach Anspruch 1, wobei
das vorbestimmte Merkmal wenigstens eines der folgenden umfasst:
- eine Anfangsspannung vor einer der Spannungsänderungen (36A-D),
- eine Endspannung nach einer der Spannungsänderungen (36A-D),
- einen Spannungsversatz zwischen einer Anfangsspannung und einer Endspannung
- eine Amplitude einer der Spannungsänderungen (36A-D),
- eine Angabe, ob die Spannungsänderung eine Spannungsabnahme oder -zunahme ist,
- eine Dauer einer der Spannungsänderungen (36A-D) und
- eine Rate einer der Spannungsänderungen (36A-D).

3. Sensoreinheit (10, 110) nach Anspruch 1 oder 2, wobei
die Steuerung (26) eingerichtet ist, um eine der Spannungsänderungen (36A-D) einem der Betriebsereignisse auf der Grundlage eines Vergleichs des vorbestimmten Merkmals mit wenigstens einem dem vorbestimmten Merkmal zugeordneten Erfassungsschwellenwert (40, 42, 44) zuzuordnen.

4. Sensoreinheit (10, 110) nach Anspruch 3, wobei
die Steuerung (26) eingerichtet ist, um eine von einem Ergebnis des Vergleichs abhängige Angabe zu erzeugen, wobei, als eine Option, die Angabe in dem Datensatz (37) enthalten ist.

5. Sensoreinheit (10, 110) nach Anspruch 3 oder 4, wobei
die Steuerung (26) eingerichtet ist, um den wenigstens einen Erfassungsschwellenwert (40, 42, 44) auf der Grundlage einer oder mehrerer der erfassten Spannungsänderungen (36A-D), die zuvor einem der Betriebsereignisse zugeordnet wurden, zu ändern.

6. Sensoreinheit (10, 110) nach einem der Ansprüche 3 bis 5, wobei
mehrere Betriebsereignisarten zum Einordnen von Betriebsereignissen definiert sind,
wobei jeder Betriebsereignisart wenigstens ein Erfassungsschwellenwert (40, 42, 44) zugeordnet ist,
und wobei die Steuerung (26) eingerichtet ist, um das Betriebsereignis einer bestimmten Betriebsereignisart auf der Grundlage des wenigstens einen vorbestimmten Merkmals und des wenigstens einen dieser Betriebsereignisart zugeordneten Erfassungsschwellenwerts (40, 42, 44) zuzuordnen.

7. Sensoreinheit (10, 110) nach Anspruch 6, wobei
die Betriebsereignisart wenigstens eine der folgenden umfasst:
- eine Aktivierung des Spenders (20),
- einen Start eines Spendeereignisses,
- einen Erholungsprozess des Spenders (20) nach einem Spendeereignis,
- eine Wiederherstellungszeit der Leistungsversorgung (21) des Spenders (20) nach einer Betätigung,
- einen Austausch des Spenders (20),
- ein Öffnen einer Abdeckung des Spenders (20) und
- eine Änderung der Leistungsversorgung (21) des Spenders (20).

8. Sensoreinheit (10, 110) nach einem der vorhergehenden Ansprüche, wobei
die Steuerung (26) eingerichtet ist, um wenigstens eine der folgenden Informationen in den Datensatz (37) aufzunehmen: eine Betriebsereignisart, einen Zeitstempel, einen Spannungsversatz zwischen einer Anfangsspannung und einer Endspannung einer der Spannungsänderungen (36A-D), eine Amplitude einer der Spannungsänderungen (36A-D) und einen Batteriestand des Spenders (20).

9. Sensoreinheit (10, 110) nach einem der vorhergehenden Ansprüche, umfassend
eine Datenschnittstelle (28), die eingerichtet ist, um den Datensatz (37) an eine Verarbeitungseinheit auszugeben.

10. Sensoreinheit (10, 110) nach einem der vorhergehenden Ansprüche, umfassend
eine Erfassungsschnittstelle (18), die eingerichtet ist, um die Sensoreinheit (10, 110) mit einer Leistungsquelle (21) des Spenders (20) zu verbinden.

11. Sensoreinheit (10, 110) nach einem der vorhergehenden Ansprüche, wobei
der Sensoreinheit (10, 110) Leistung von einer Leistungsquelle (21) des Spenders (20) zugeführt wird.

12. Sensoreinheit (10, 110) nach einem der vorhergehenden Ansprüche, umfassend
eine Leistungsquelle (14), die eingerichtet ist, um die Sensoreinheit (10, 110) mit Leistung zu versorgen.

13. Sensoreinheit (10) nach einem der vorhergehenden Ansprüche, ferner umfassend
eine Signalisierungseinheit (16), die eingerichtet ist, um einem Benutzer eines der Betriebsereignisse und/oder zusätzliche Informationen zu signalisieren.

14. System umfassend
die Sensoreinheit (10, 110) nach einem der vorhergehenden Ansprüche; und
einen automatischen Spender (20), mit dem die Sensoreinheit (10, 110) derart gekoppelt ist, dass sie eingerichtet ist, um eine Änderung einer Betriebsspannung des Spenders (20) zu erfassen.

15. System nach Anspruch 14, wobei
der Spender (20) ein Desinfektionsmittelspender, ein Seifenspender, ein Handpflegespender oder ein Reinigungsmittelspender ist.

16. Verfahren zum Erfassen von Betriebsereignissen eines automatischen Spenders (20), wobei die Betriebsereignisse Spendeereignisse umfassen, und wobei das Verfahren umfasst:
- Erfassen (S4) einer Folge von Änderungen (36A-D) einer Betriebsspannung des Spenders (20);
- Analysieren (S5) der Spannungsänderungen (36A-D) hinsichtlich vorbestimmter Merkmale, um eine Spannungsänderung (36C) zu erfassen, die mit einem Spendeereignis des Spenders (20) zusammenhängt;
- Zuordnen (S6) einer der erfassten Spannungsänderungen (36B, 36C, 36D) zu einem Spendeereignis des Spenders (20) auf der Grundlage der Analyse,
wobei eine zweite Spannungsänderung (36B), die einer dritten mit dem Spendeereignis zusammenhängenden Spannungsänderung (36C) vorausgeht, einen Start eines Elektromotors des Spenders (20) angibt und einem Start des Spendeereignisses auf der Grundlage einer Spannungsabnahme, die eine Amplitude aufweist, die innerhalb einer bestimmten Zeitspanne unter einen ersten Erfassungsschwellenwert (40) fällt, als ein vorbestimmtes Merkmal zugeordnet ist,
wobei die dritte, mit dem Spendeereignis zusammenhängende Spannungsänderung (36C) eine längere Zeitspanne andauert und eine geringere Energiemenge benötigt als die vorhergehende zweite Spannungsänderung (36B) und dem Spendeereignis, bei dem der Elektromotor wiederholt ein mechanisches Element des Spenders (20) bewegt, auf der Grundlage eines kontinuierlichen Anstiegs der Spannung als ein vorbestimmtes Merkmal zugeordnet ist, und
wobei die Zeitspanne der dritten Spannungsänderung (36C), die mit dem Spendeereignis zusammenhängt, mit der Erfassung einer nachfolgenden vierten Spannungsänderung (36D) endet, während welcher der Elektromotor über einen kürzeren Zeitraum weniger Energie verbraucht als während der dritten Spannungsänderung (36C), die mit dem Spendeereignis zusammenhängt, wobei die nachfolgende vierte Spannungsänderung (36D) einem Erholungsprozess des Spenders (20) auf der Grundlage einer Amplitude des Spannungsanstiegs als ein vorbestimmtes Merkmal zugeordnet ist; und
- Erzeugen (S7) eines Datensatzes (37), der das Spendeereignis und die Zeitspanne der dritten Spannungsänderung (36C) im Zusammenhang mit dem Spendeereignis angibt.

17. Computerprogrammprodukt mit Programmcodeteilen zur Durchführung der Schritte des Verfahrens nach Anspruch 16, wenn das Computerprogrammprodukt auf einem oder mehreren Prozessoren ausgeführt wird.

## Revendications

1. Unité (10, 110) de capteur configurée pour détecter des événements de fonctionnement d'un distributeur automatique (20), les événements de fonctionnement comprenant des événements de distribution, l'unité (10, 110) de capteur comprenant :
une unité (24) de détection configurée pour détecter une séquence de changements (36A-D) d'une tension de fonctionnement du distributeur (20) ; et
un contrôleur (26) configuré pour :
- analyser les changements de tension (36A-D) pour des caractéristiques prédéterminées pour détecter un changement de tension (36C) qui est lié à un événement de distribution du distributeur (20) ;
- attribuer un des changements de tension (36B, 36C, 36D) détectés à un événement de distribution du distributeur (20) sur la base de l'analyse,
dans laquelle un deuxième changement de tension (36B) précédant un troisième changement de tension (36C) qui est lié à l'événement de distribution est indicateur d'un démarrage d'un moteur électrique du distributeur (20) et est attribué à un démarrage de l'événement de distribution sur la base d'une diminution de tension ayant une amplitude tombant en-dessous d'un premier seuil de détection (40) sur une certaine période de temps comme une caractéristique prédéterminée,
dans laquelle le troisième changement de tension (36C) lié à l'événement de distribution dure une longueur de temps plus longue et requiert une quantité d'énergie moindre que le deuxième changement de tension (36B) précédent et est attribué à l'événement de distribution pendant lequel le moteur électrique déplace de façon répétée un élément mécanique du distributeur (20) sur la base d'une augmentation continue de la tension comme une caractéristique prédéterminée, et
dans laquelle la longueur de temps du troisième changement de tension (36C) lié à l'événement de distribution se termine avec une détection d'un quatrième changement de tension (36D) suivant pendant lequel le moteur électrique consomme une énergie moindre sur une période de temps plus courte que pendant le troisième changement de tension (36C) lié à l'événement de distribution, dans laquelle le quatrième changement de tension (36D) suivant est attribué à un processus de récupération du distributeur (20) sur la base d'une amplitude de l'augmentation de tension comme une caractéristique prédéterminée ; et
- créer un ensemble de données (37) indicateur de l'événement de distribution et de la longueur de temps du troisième changement de tension (36C) lié à l'événement de distribution.

2. Unité (10, 110) de capteur selon la revendication 1, dans laquelle la caractéristique prédéterminée comprend au moins une parmi les suivantes :
- une tension initiale avant un des changements de tension (36A-D),
- une tension de fin après un des changements de tension (36A-D),
- un décalage de tension entre une tension initiale et une tension de fin,
- une amplitude d'un des changements de tension (36A-D),
- une indication si le changement de tension est une diminution ou une augmentation de tension,
- une durée d'un des changements de tension (36A-D), et
- une vitesse d'un des changements de tension (36A-D).

3. Unité (10, 110) de capteur selon la revendication 1 ou 2, dans laquelle
le contrôleur (26) est configuré pour attribuer un des changements de tension (36A-D) à un des événements de fonctionnement sur la base d'une comparaison de la caractéristique prédéterminée avec au moins un seuil de détection (40, 42, 44) associé avec la caractéristique prédéterminée.

4. Unité (10, 110) de capteur selon la revendication 3, dans laquelle
le contrôleur (26) est configuré pour créer une indication en fonction d'un résultat de la comparaison, dans laquelle, facultativement, l'indication est incluse dans l'ensemble de données (37).

5. Unité (10, 110) de capteur selon la revendication 3 ou 4, dans laquelle le contrôleur (26) est configuré pour modifier l'au moins un seuil de détection (40, 42, 44) sur la base d'un ou de plusieurs parmi les changements de tension (36AD) détectés qui ont été auparavant attribués à un des événements de fonctionnement.

6. Unité (10, 110) de capteur selon l'une quelconque des revendications 3 à 5, dans laquelle
des types multiples d'événements de fonctionnement sont définis pour catégoriser des événements de fonctionnement,
dans laquelle chaque type d'événement de fonctionnement est associé avec au moins un seuil de détection (40, 42, 44),
et dans laquelle le contrôleur (26) est configuré pour attribuer l'événement de fonctionnement à un type d'événement de fonctionnement particulier sur la base de l'au moins une caractéristique prédéterminée et de l'au moins un seuil de détection (40, 42, 44) associé avec ce type d'événement de fonctionnement.

7. Unité (10, 110) de capteur selon la revendication 6, dans laquelle
le type d'événement de fonctionnement comprend au moins un(e) des suivant(e)s :
- une activation du distributeur (20),
- un démarrage d'un événement de distribution,
- un processus de récupération du distributeur (20) après un événement de distribution,
- un temps de récupération de l'alimentation électrique (21) du distributeur (20) après actionnement,
- un remplacement du distributeur (20),
- une ouverture d'un capot du distributeur (20), et
- un changement de l'alimentation électrique (21) du distributeur (20).

8. Unité (10, 110) de capteur selon l'une quelconque des revendications précédentes, dans laquelle
le contrôleur (26) est configuré pour inclure au moins un des éléments d'information suivants dans l'ensemble de données (37) : un type d'événement de fonctionnement, un horodatage, un décalage de tension entre une tension initiale et une tension de fin d'un des changements de tension (36A-D), une amplitude d'un des changements de tension (36A-D), et un niveau de batterie du distributeur (20).

9. Unité (10, 110) de capteur selon l'une quelconque des revendications précédentes, comprenant
une interface de données (28) configurée pour délivrer en sortie l'ensemble de données (37) jusqu'à une unité de traitement.

10. Unité (10, 110) de capteur selon l'une quelconque des revendications précédentes, comprenant
une interface de détection (18) configurée pour connecter l'unité (10, 110) de capteur à une source d'alimentation électrique (21) du distributeur (20).

11. Unité (10, 110) de capteur selon l'une quelconque des revendications précédentes, dans laquelle
l'alimentation électrique est fournie à l'unité (10, 110) de capteur depuis une source d'alimentation électrique (21) du distributeur (20).

12. Unité (10, 110) de capteur selon l'une quelconque des revendications précédentes, comprenant
une source d'alimentation électrique (14) configurée pour fournir une énergie électrique à l'unité (10, 110) de capteur.

13. Unité (10, 110) de capteur selon l'une quelconque des revendications précédentes, comprenant
une unité (16) de signalisation configurée pour signaler à un utilisateur un des événements de fonctionnement et/ou des informations additionnelles.

14. Système comprenant
l'unité (10, 110) de capteur selon l'une quelconque des revendications précédentes ; et
un distributeur automatique (20) auquel l'unité (10, 110) de capteur est couplée de façon à être configurée pour détecter un changement d'une tension de fonctionnement du distributeur (20).

15. Système selon la revendication 14, dans lequel
le distributeur (20) est un distributeur de désinfectant, un distributeur de savon, un distributeur de produit de soin des mains ou un distributeur d'agent de nettoyage.

16. Procédé pour détecter des événements de fonctionnement d'un distributeur automatique (20), les événements de fonctionnement comprenant des événements de distribution, le procédé comprenant :
- la détection (S4) d'une séquence de changements (36A-D) d'une tension de fonctionnement du distributeur (20) ;
- l'analyse (S5) des changements de tension (36A-D) pour des caractéristiques prédéterminées pour détecter un changement de tension (36C) qui est lié à un événement de distribution du distributeur (20) ;
- l'attribution (S6) d'un des changements de tension (36B, 36C, 36D) détectés à un événement de distribution du distributeur (20) sur la base de l'analyse,
dans lequel un deuxième changement de tension (36B) précédant un troisième changement de tension (36C) qui est lié à l'événement de distribution est indicateur d'un démarrage d'un moteur électrique du distributeur (20) et est attribué à un démarrage de l'événement de distribution sur la base d'une diminution de tension ayant une amplitude tombant en-dessous d'un premier seuil de détection (40) sur une certaine période de temps comme une caractéristique prédéterminée,
dans lequel le troisième changement de tension (36C) lié à l'événement de distribution dure une longueur de temps plus longue et requiert une quantité d'énergie moindre que le deuxième changement de tension (36B) précédent et est attribué à l'événement de distribution pendant lequel le moteur électrique déplace de façon répétée un élément mécanique du distributeur (20) sur la base d'une augmentation continue de la tension comme une caractéristique prédéterminée, et
dans lequel la longueur de temps du troisième changement de tension (36C) lié à l'événement de distribution se termine avec une détection d'un quatrième changement de tension (36D) suivant pendant lequel le moteur électrique consomme une énergie moindre sur une période de temps plus courte que pendant le troisième changement de tension (36C) lié à l'événement de distribution, dans lequel le quatrième changement de tension (36D) suivant est attribué à un processus de récupération du distributeur (20) sur la base d'une amplitude de l'augmentation de tension comme une caractéristique prédéterminée ; et
- la création (S7) d'un ensemble de données (37) indicateur de l'événement de distribution et de la longueur de temps du troisième changement de tension (36C) lié à l'événement de distribution.

17. Produit de programme informatique comprenant des parties de code de programme pour mettre en œuvre les étapes du procédé selon la revendication 16 lorsque le produit de programme informatique est exécuté sur un ou plusieurs processeurs.
